# EUROPEAN PATENT APPLICATION

(11) **EP 4 279 508 A1**
(43) Date of publication of application: **22.11.2023**
(21) Application number: 22739063.0
(22) Date of filing: 12.01.2022
(51) Int. Cl.: C07K 16/28, C07K 16/46, C12N 15/13, A61K 39/395, A61P 35/00

(54) **CD5-TARGETING FULLY HUMANIZED ANTIBODY**

(30) Priority: 12.01.2021 CN 202110032708
(71) Applicant: Nanjing Iaso Biotechnology Co., Ltd., Nanjing, Jiangsu 210000 (CN)
(72) Inventor: TAN, Taochao, Nanjing, Jiangsu 210000 (CN); WEI, Qiaoe, Nanjing, Jiangsu 210000 (CN); JIA, Xiangyin, Nanjing, Jiangsu 210000 (CN); TAN, Jiayue, Nanjing, Jiangsu 210000 (CN); XIE, Meng, Nanjing, Jiangsu 210000 (CN); DAI, Zhenyu, Nanjing, Jiangsu 210000 (CN)
(74) Representative: Symbiosis IP Limited
(86) International application number: PCT/CN2022/071674
(87) International publication number: WO 2022/152185

(57) **Abstract**

Provided is a CDS-targeting fully human antibody or an antigen-binding fragment thereof, which specifically binds to CD5 with a high affinity, has a lower immunogenicity compared to heterologous antibodies, and has a good application potential in the development of antibody drugs, cell therapy drugs, detection reagents and the like.

## Description

### CROSS-REFERENCE TO RELATED APPLICATION

The present application claims priority to Chinese Patent Application No. 202110032708.5 filed with the China Patent Office on January 12, 2021, which is hereby incorporated by reference in its entirety.

### FIELD OF THE INVENTION

The invention relates to a fully human antibody or an antigen-binding fragment thereof that specifically binds to human CD5 antigen protein and CD5 antigen in its natural state on the surface of the cell membrane.

### BACKGROUND OF THE INVENTION

At present, the clinical cure of patients with T-cell malignancies is still poor, and there is no better treatment strategy than chemotherapy. But chemotherapy has no benefit for relapsed and refractory patients, and has serious side effects. Therefore, it is imperative to develop novel and effective targeted therapeutic strategies. In recent years, chimeric antigen receptor (CAR)-modified immune cells and antibody drugs have shown outstanding efficacy in the treatment of B-cell malignancies. By analogy, the development of CAR-modified immune cells or antibody drugs may contribute to the treatment of T-cell malignancies.

CD5 is a type I transmembrane glycosylated protein that plays an important role in the negative regulation of T-cell receptor signaling and promotes the survival of normal and malignant lymphocytes. CD5 is not expressed on the surface of hematopoietic stem cells, but is highly expressed on malignant T-cells. CD5 is one of the characteristic surface markers of malignant T-cell tumors, and 80% of T-cell acute lymphoblastic leukemia (T-ALL) and peripheral T-cell lymphomas express CD5. In addition, CD5 is also expressed on some malignant B-cell tumors. In clinical trials using CD5 mAbs, CD5 mAbs have shown moderate therapeutic efficacy in patients with cutaneous T-cell lymphoma (CTCL) or chronic lymphocytic leukemia (CLL).

Therefore, the development of fully human antibodies that can exert clinically effective cytotoxic, cytostatic or immunosuppressive effects on cells expressing CD5, and have no adverse effects on cells that do not express CD5, is of great significance for the development of immunotherapy products related to CD5 expression.

### SUMMARY OF THE INVENTION

In one aspect, provided herein is a CD5-targeting antibody or an antigen-binding fragment thereof, wherein the heavy chain variable region of the antibody comprises HCDR1, HCDR2, and HCDR3, and the HCDR1, HCDR2, and HCDR3 are selected from one of the following combinations:
(1) the amino acid sequence of HCDR1 is GFTFSHSA (SEQ ID NO: 1);
   the amino acid sequence of HCDR2 is IYARGGYT (SEQ ID NO: 2);
   the amino acid sequence of HCDR3 is ARGYHLEYMVSQDV (SEQ ID NO: 3);
(2) the amino acid sequence of HCDR1 is GFTFSSYE (SEQ ID NO: 4);
   the amino acid sequence of HCDR2 is ISSSGSTI (SEQ ID NO: 5);
   the amino acid sequence of HCDR3 is ARVAQREGDV (SEQ ID NO: 6);
(3) the amino acid sequence of HCDR1 is GGTFSNYA (SEQ ID NO: 7);
   the amino acid sequence of HCDR2 is ISAYNGDT (SEQ ID NO: 8);
   the amino acid sequence of HCDR3 is ARYESMSGQDI (SEQ ID NO: 9);
(4) the amino acid sequence of HCDR1 is GYSFSNHW (SEQ ID NO: 10);
   the amino acid sequence of HCDR2 is VYPGDSDT (SEQ ID NO: 11);
   the amino acid sequence of HCDR3 is ARGGTIDGDYGGRQDF (SEQ ID NO: 12); or
   the antibody comprises a variant of the combination of CDR sequences in any one of (1)-(4), wherein compared to the CDR sequences in any one of (1)-(4), the variant has at least 90% sequence identity, or comprise a total of at least 1 and no more than 10, or no more than 5, 4, 3, 2 or 1 amino acid changes in the CDR sequences.

In some embodiments, the amino acid sequence of the heavy chain variable region is selected from any of the following:
(1) an amino acid sequence as set forth in SEQ ID NO: 17 or a heavy chain variable region having at least 90% sequence identity therewith;
(2) an amino acid sequence as set forth in SEQ ID NO: 18 or a heavy chain variable region having at least 90% sequence identity therewith;
(3) an amino acid sequence as set forth in SEQ ID NO: 19 or a heavy chain variable region having at least 90% sequence identity therewith;
(4) an amino acid sequence as set forth in SEQ ID NO: 20 or a heavy chain variable region having at least 90% sequence identity therewith.

In some embodiments, the amino acid sequence of the heavy chain variable region and/or light chain variable region is selected from any of the following:
(1) a heavy chain variable region sequence as set forth in SEQ ID NO: 17;
(2) a heavy chain variable region sequence as set forth in SEQ ID NO: 18;
(3) a heavy chain variable region sequence as set forth in SEQ ID NO: 19;
(4) a heavy chain variable region sequence as set forth in SEQ ID NO: 20.

In some embodiments, the antibody is a fully human antibody.

In some embodiments, the antibody is a single-domain antibody.

In some embodiments, the binding KD value of the antibody to CD5 antigen determined by biolayer interferometry is lower than 10⁻⁷ M, preferably lower than 10⁻⁸ M.

In another aspect, the present application also provides a fusion protein, which comprises one or two antigen-binding functional moieties, wherein each of the antigen-binding functional moieties comprises the above-mentioned antibody or antigen-binding fragment thereof.

In some embodiments, the fusion protein further comprises an Fc fragment.

In some embodiments, the two antigen-binding functional moieties respectively bind to the same or different antigenic epitopes.

In some embodiments, the fusion protein comprises a first antigen-binding functional moiety and a second antigen-binding functional moiety connected in tandem; wherein the first antigen-binding functional moiety comprises a first heavy chain variable region (HCVR), and the first heavy chain variable region comprises HCDR1 as set forth in SEQ ID NO: 7, HCDR2 as set forth in SEQ ID NO: 8 and HCDR3 as set forth in SEQ ID NO: 9; wherein the second antigen binding functional moiety comprises a second heavy chain variable region (HCVR), and the second heavy chain variable region comprises HCDR1 as set forth in SEQ ID NO: 1, HCDR2 as set forth in SEQ ID NO: 2, and HCDR3 as set forth in SEQ ID NO: 3.

In some embodiments, the first antigen-binding functional moiety is at N-terminal of the second antigen-binding functional moiety.

In some embodiments, the fusion protein comprises the heavy chain variable region sequence as set forth in SEQ ID NO: 19 and the heavy chain variable region sequence as set forth in SEQ ID NO: 17 connected in tandem.

In some embodiments, the heavy chain variable region sequence as set forth in SEQ ID NO: 19 is at N-terminal of the heavy chain variable region sequence as set forth in SEQ ID NO: 17.

In some embodiments, the antigen-binding functional moieties are directly connected through a linker molecule; preferably, the linker molecule comprises an amino acid sequence as set forth in SEQ ID NO: 21.

In some embodiments, for the fusion protein, the EC₅₀ value of the binding between the fusion protein and CD5 positive cells determined by flow cytometry is 1-5 nM.

In another aspect, the present application further includes an isolated nucleic acid molecule encoding the above-mentioned antibody or antigen-binding fragment thereof or the fusion protein.

In some embodiments, the nucleic acid molecule comprises a nucleotide sequence as set forth in any one of SEQ ID NOs: 13-16.

In another aspect, the present application further includes an expression vector comprising the nucleic acid molecule of the present application. In some embodiments, the vector is a plasmid, a retroviral vector and a lentiviral vector.

In another aspect, the present application further includes a host cell comprising the expression vector of the present application.

In another aspect, the present application further includes a pharmaceutical composition, which comprises the antibody or antigen-binding fragment thereof or the fusion protein of the present application, and a pharmaceutically acceptable carrier or diluent.

In another aspect, the present application further includes a method of treating a disease or condition by administering to a patient in need thereof a therapeutically effective amount of the antibody or antigen-binding fragment thereof, the fusion protein, or the host cell, or the pharmaceutical composition of the present application to eliminate, inhibit or reduce CD5 activity, thereby preventing, alleviating, ameliorating or inhibiting the disease or condition.

The present application further includes the use of the above-mentioned antibody or antigen-binding fragment thereof, the fusion protein or the above-mentioned host cell in the preparation of a drug for eliminating, inhibiting or reducing CD5 activity, thereby preventing, alleviating, ameliorating or inhibiting a disease or condition.

The present application further includes the above-mentioned antibody or antigen-binding fragment thereof, the fusion protein or the above-mentioned host cell for use as a drug or in treatment, for example, for eliminating, inhibiting or reducing CD5 activity, thereby preventing, alleviating, ameliorating or inhibiting a disease or condition.

In some embodiments, the disease or condition is selected from: cancers or autoimmune diseases. In some embodiments, the cancer is selected from: malignant T-cell tumors or malignant B-cell tumors T-cell malignant tumor.

In some embodiments, the malignant T-cell tumor is selected from T-cell acute lymphoblastic leukemia (T-ALL), T-cell lymphoma (such as: peripheral T-cell lymphoma or cutaneous T-cell lymphoma (CTCL)), and the malignant B-cell tumor is selected from chronic lymphocytic leukemia (B-CLL) or mantle cell lymphoma (B-MCL).

In another aspect, the present application further includes a kit for detecting CD5 protein in a sample. The kit includes the antibody or antigen-binding fragment thereof or fusion protein in the present application. The detection can be in vitro or in vivo.

In another aspect, the present application further includes an antibody or fragment competing for the same epitope as the antibody or antigen-binding fragment thereof of the present application.

In another aspect, the present application further includes a multispecific antibody molecule, which comprises at least a first functional moiety and a second functional moiety, wherein the first functional moiety comprises the antibody or antigen-binding fragment thereof of the present application; and the second functional moiety has different binding specificity than the first functional moiety.

In some embodiments, the second functional moiety has binding specificity for immune cells.

In some embodiments, the second functional moiety has binding specificity for T-cells.

In some embodiments, the second functional moiety has binding specificity for CD7.

In another aspect, the present application further includes an immunoconjugate, which comprises the antibody or antigen-binding fragment thereof of any one of claims 1-6 linked to a therapeutic agent.

In some embodiments, the therapeutic agent is a drug.

In some embodiments, the therapeutic agent is a cytotoxin.

In some embodiments, the therapeutic agent is a radioisotope.

In another aspect, the present application further includes the use of the above-mentioned antibody or antigen-binding fragment thereof, the above-mentioned fusion protein, the above-mentioned multispecific antibody molecule or the above-mentioned immunoconjugate in the preparation of a drug for eliminating, inhibiting or reducing CD5 activity.

In some embodiments, the drug is used for preventing, alleviating, ameliorating or inhibiting cancers or autoimmune diseases.

In some embodiments, the cancer is selected from: malignant T-cell tumors or malignant B-cell tumors.

In another aspect, the present application further includes the use of the above-mentioned antibody or antigen-binding fragment thereof, the above-mentioned fusion protein, the above-mentioned multispecific antibody molecule or the above-mentioned immunoconjugate in the preparation of a vaccine, preferably an antibody vaccine, and more preferably an anti-idiotypic antibody vaccine.

In another aspect, the present application further includes a vaccine preparation, which comprises the above-mentioned antibody or antigen-binding fragment thereof, the above-mentioned fusion protein, the above-mentioned multispecific antibody molecule or the above-mentioned immunoconjugate.

Those skilled in the art can easily perceive other aspects and advantages of the present application from the following detailed description. In the following detailed description, only exemplary embodiments of the present application are shown and described. As those skilled in the art will appreciate, the content of the present application enables those skilled in the art to make changes to the disclosed specific embodiments without departing from the spirit and scope of the invention to which the present application relates. Correspondingly, the drawings and descriptions in the specification of the present application are only exemplary rather than restrictive.

### DESCRIPTION OF DRAWINGS

Fig. 1 shows the general flow of the present invention for screening specific antibodies targeting CD5 from a phage antibody library.
Fig. 2 shows the results of enzyme-linked immunosorbent assay (ELISA) for some of the panned phage monoclonal bodies with target antigens and control antigens.
Fig. 3 shows the results of flow cytometry for the binding of some phage monoclonal bodies to Raji and Jurkat cells.
Figs. 4 A-F show the results of flow cytometry (peak pattern and MFI value) of the screened phage monoclonal bodies #1-64 binding to various CD5 positive and negative cell lines. Negative Control is a negative control phage antibody clone.

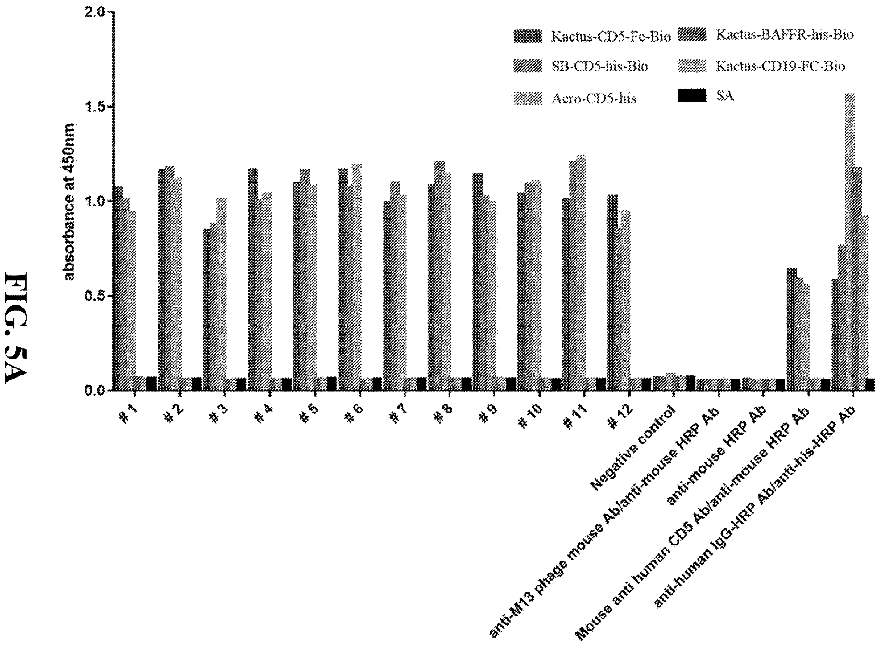

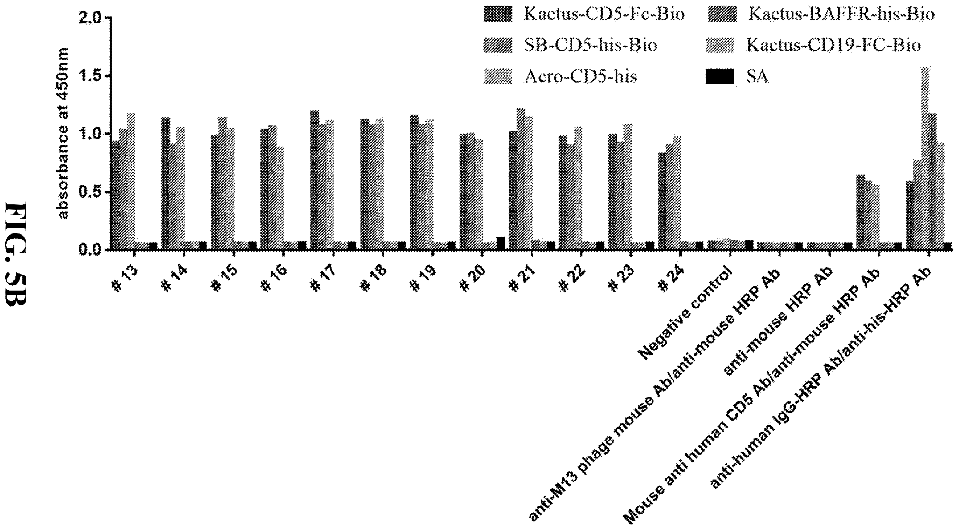

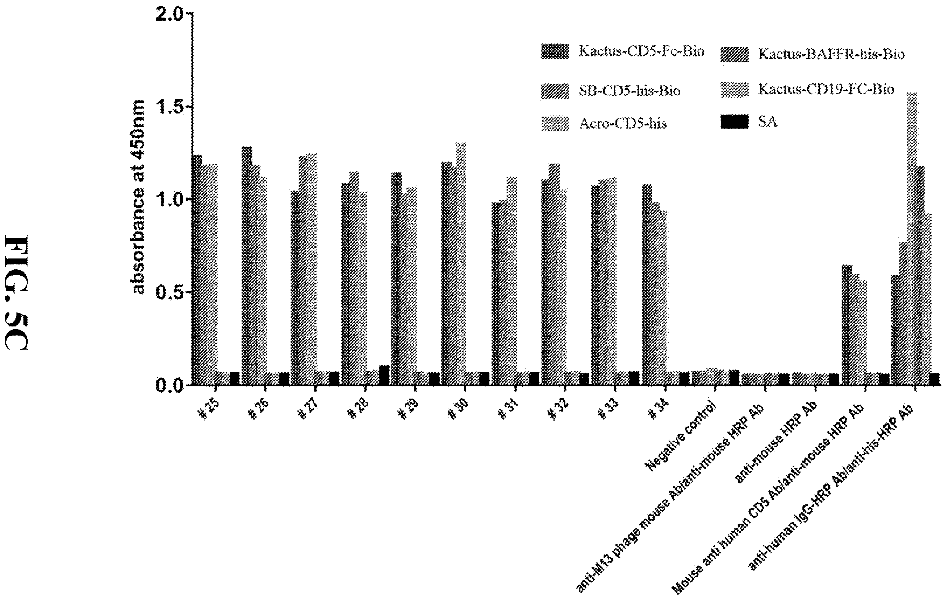

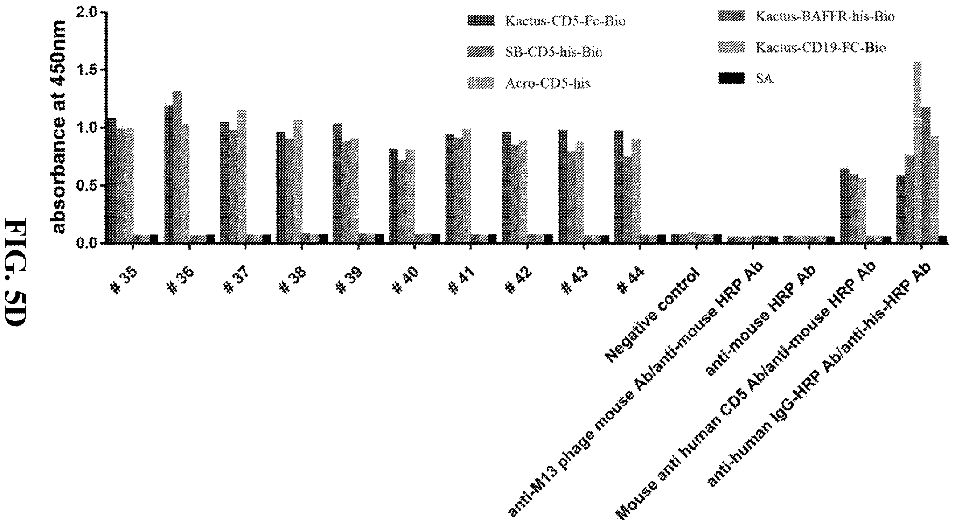

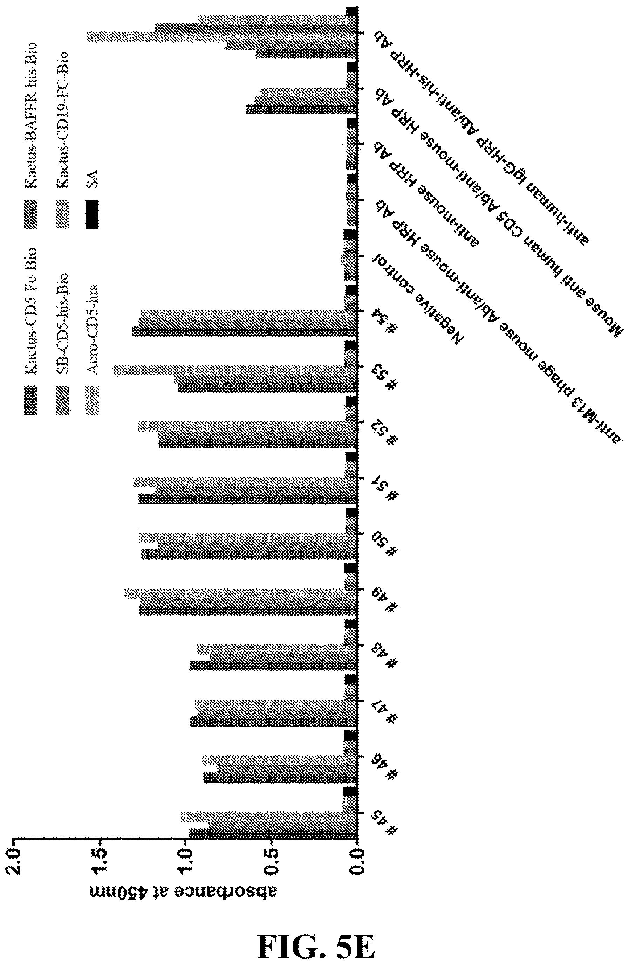

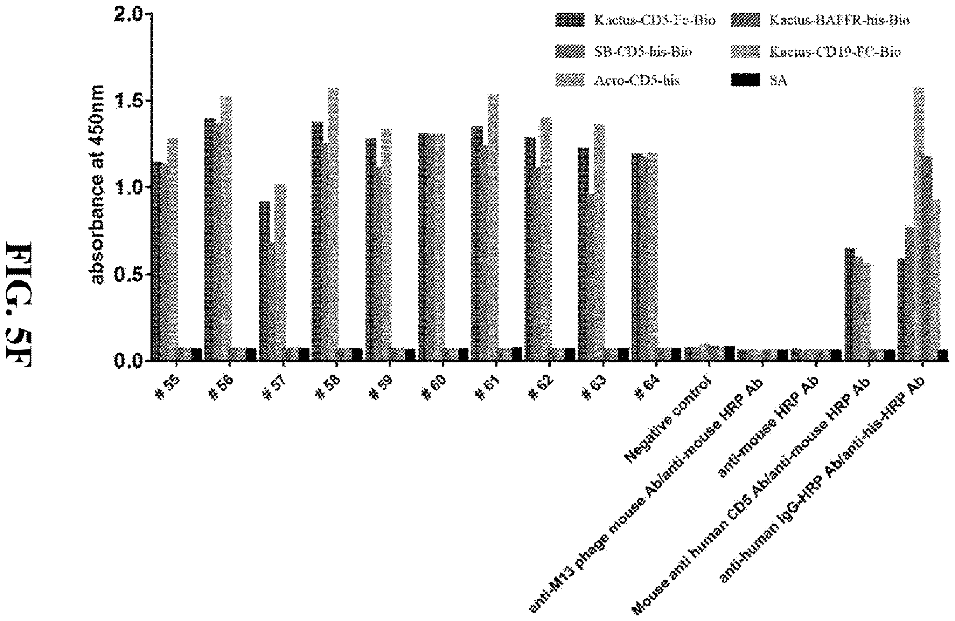

Figs. 5 A-F show the results of ELISA analysis of the screened phage monoclonal bodies #1-64 with CD5 antigen proteins from various companies and non-related antigens. Negative control is a negative control phage antibody clone, anti-M13 phage mouse Ab/anti-mouse HRP Ab is a negative antibody control with only primary and secondary antibodies added, and anti-mouse HRP Ab is a negative antibody control with only secondary antibodies added, mouse anti human CD5 Ab/anti-mouse HRP Ab is the positive antibody control for the target antigen (CD5-Fc-Bio), anti-human IgG-HRP Ab/anti-his-HRP Ab is the positive antibody control for detecting the antigen label. Among them, in Figs. 5A-F, the histograms corresponding to the respective test antibodies and the control group indicate from left to right the test results of proteins Kactus-CD5-Fc-Bio, SB-CD5-his-Bio, Acro-CD5-his, Kactus-BAFFR-his-Bio, Kactus-CD19-FC-Bio, and SA.
Fig. 6 shows the results of the study on the binding of RD125 61-42-rFc single-domain antibody rabbit Fc fusion protein to CD5 positive cells.

### DETAILED DESCRIPTION OF THE INVENTION

Unless otherwise defined, all technical and scientific terms used herein have the meaning as commonly understood by one of ordinary skill in the art.

"Antibody" refers to an immunoglobulin secreted by plasma cells (effector B-cells) and used by the body's immune system to neutralize foreign substances (polypeptides, viruses, bacteria, etc.). The foreign substance is correspondingly called an antigen. The basic structure of a classical antibody molecule is a 4-mer consisting of 2 identical heavy chains and 2 identical light chains. According to the conservative differences in amino acid sequences, the heavy and light chains are divided into a variable region (V) at the amino terminus and a constant region (C) at the carboxy terminus. The variable regions of one heavy chain and one light chain interact to form the antigen-binding site (Fv). In the variable region, the composition and arrangement of amino acid residues in certain regions are more variable than other regions (framework regions, FRs) in the variable region, these regions are called hypervariable regions (HVRs) and are actually the key sites for binding of antibodies to antigens. Since these hypervariable regions have their sequences complementary to antigenic determinants, they are also called complementarity-determining regions (CDRs). Both heavy and light chains have three complementarity-determining regions, designated HCDR1, HCDR2, HCDR3 and LCDR1, LCDR2, LCDR3, respectively.

"Single chain fragment variable (scFv)" is composed of antibody heavy and light chain variable regions linked by a short peptide into a peptide chain. Through correct folding, the variable regions from the heavy chain and the light chain interact through non-covalent bonds to form Fv segments, so scFv can better retain its affinity activity for antigens.

"Single-domain antibody" refers to an antibody composed only of the variable region amino acids of a heavy chain antibody. Its molecular weight is only 12-15 kDa, but it has similar or higher specificity and affinity than traditional antibodies. In addition, single-domain antibodies have attracted much attention because of their stable physical and chemical properties, high affinity, easy recombinant expression and preparation, and easy combination with other target or epitope antibodies.

"Murine antibody" is an antibody produced by murine against a specific antigen, usually referring to an antibody produced by mouse B lymphocytes. In most cases, the murine antibody is a monoclonal antibody produced by hybridoma cells. The fully human antibody of the present application is obtained by screening a human phage antibody library, which has reduced immunogenicity compared to the murine antibody, and is more conducive to the therapeutic use in the human body.

The "antibody or antigen-binding fragment thereof" of the present application generally refers to any form of antigen-binding molecule capable of binding to a target antigen, for example, the antigen-binding molecule can be a protein or a polypeptide, including for example antibodies and antigen-binding fragments thereof, single-chain scFv antibodies, single-domain antibodies, various fusions and conjugates constructed based on scFv, such as scFv-Fc antibodies, immunoconjugates, antibody drug conjugates (ADCs), multi/bispecific antibodies, chimeric antigen receptors (CARs).

CD5 is a type I transmembrane glycosylated protein that plays an important role in the negative regulation of T-cell receptor signaling and promotes the survival of normal and malignant lymphocytes. CD5 is one of the characteristic surface markers of malignant T-cell tumors, and 80% of T-cell acute lymphoblastic leukemia (T-ALL) and peripheral T-cell lymphomas express CD5. This forms the basis for the clinical application of antibodies targeting CD5 in the treatment of relevant tumors.

The term "sequence identity" when referring to amino acid or nucleotide sequences refers to the degree of identity between two amino acid or nucleotide sequences (e.g., a query sequence and a reference sequence), usually expressed as a percentage. Typically, prior to calculating the percent identity between two amino acid or nucleotide sequences, the sequences are aligned and gaps, if any, introduced. If at a certain alignment position, the amino acid residues or bases in the two sequences are the same, the two sequences are considered to be identical or matched at that position; and if the amino acid residues or bases in the two sequences are different, they are considered to be non-identical or mismatched at that position. In some algorithms, the number of matched positions is divided by the total number of positions in the alignment window to obtain sequence identity. In other algorithms, the number of gaps and/or the gap length are also taken into account. For the purposes of the present invention, the published alignment software BLAST (available at ncbi.nlm.nih.gov) can be employed to obtain optimal sequence alignments by using default settings and calculate the sequence identity between two amino acid or nucleotide sequences. In some embodiments, "at least 90% sequence identity" as mentioned in the present application includes, but is not limited to: at least 95%, at least 98%, at least 99% or even 100% sequence identity.

Those skilled in the art can understand that, on the basis of the specific sequences provided herein, corresponding variants of the antibody targeting CD5 provided herein can be obtained by substituting, deleting, adding a few amino acids, and verifying or screening the resultant product for its binding ability with the corresponding antigen CD5 or its biological activity, and these variants should also be included within the scope of the present invention. For example, the fully human antibody or antigen-binding fragment thereof of the present application may have at least 1 and no more than 10, or no more than 5, 4, 3, 2 or 1 amino acid changes in the full length or CDR sequence.

Those skilled in the art can also understand that, on the basis of the specific heavy chain variable region sequences provided herein, an antibody light chain library (such as a human phage light chain library) can be screened by using CD5 as the antigen, so as to obtain light chain variable regions matched with the heavy chain variable region while maintaining CD5 binding ability. Anti-CD5 antibody molecules obtainable in this way are also included in the scope of the present invention.

In some embodiments, the antigen binding molecules of the present application may further comprise post-translational modifications. Examples of post-translational protein modifications include: phosphorylation, acetylation, methylation, ADP-ribosylation, ubiquitination, glycosylation, carbonylation, SUMOylation, biotinylation, or addition of polypeptide side chains or hydrophobic groups. Thus, a modified soluble polypeptide may comprise non-amino acid components such as lipoids, polysaccharides or monosaccharides, and phosphates. A preferred form of glycosylation is sialylation modification, which binds one or more sialic acid groups to polypeptides. The sialic acid group improves the solubility and serum half-life of the protein, while also reducing the possible immunogenetic property of the protein. See Raju et al. Biochemistry. 2001 31; 40(30):8868-76.

When referring to pharmaceutical compositions, "pharmaceutically acceptable carrier" is used to refer to substances such as solid or liquid diluents, fillers, antioxidants, and stabilizers, which are safe for administration, and which are suitable for administration to humans and/or animals without undue adverse side effects, while being suitable for maintaining the viability of the drug or active agent therein.

A "therapeutically effective amount" refers to an amount of an active compound sufficient to elicit the biological or medical response desired by a clinician in a subject. The "therapeutically effective amount" of the antibody of the present application can be determined by those skilled in the art according to the administration route, the subject's body weight, age, condition and other factors. For example, a typical daily dose may range from 0.01 mg to 100 mg of active ingredient per kg of body weight. The administration mode of the antibody of the present application includes but is not limited to injection, such as by intravenous, intramuscular, intraarterial, subcutaneous, intraperitoneal injection and the like.

"Epitope" refers to the portion of a molecule that is bound by an antigen binding protein (e.g., antibody). Epitopes can comprise non-adjacent portions of the molecule (e.g., in a polypeptide, amino acid residues that are not adjacent in the main sequence of the polypeptide, but are close enough to each other in the trivalent and tetravalent structures of the polypeptide to be bound by antigen-binding proteins).

"Fusion protein" refers to a protein molecule that is artificially produced (e.g., through genetic engineering techniques) and consists of at least two different peptide segments. These peptide segments do not exist in nature, or do not exist in the same protein molecule. Common examples of fusion proteins that comprise antibody fragments include antibody-cytokine fusion proteins, antibody-cytotoxin fusion proteins (also known as immunotoxins), enzyme-labeled antibodies for immunoassays, chimeric antigen receptors (CARs) etc. In a specific example, a fusion protein can comprise at least two single-domain antibodies provided herein, and the two single-domain antibodies can bind to the same or different antigenic epitopes.

KD value can be used to measure the binding affinity between an antibody and its antigen. The KD value is the equilibrium dissociation constant between an antibody and its antigen, that is, the ratio of k_{off}/kₒₙ. Thus, the lower the KD value (the lower the concentration), the higher the affinity of the antibody.

EC₅₀ (concentration for 50% of maximal effect) refers to the concentration that causes 50% of the maximal effect. When used in flow cytometry to indicate the binding ability of antibody molecules to corresponding antigens or cells expressing antigens, it can refer to the concentration of antibody molecules that produces half of the maximal detection signal (such as fluorescence intensity). The lower the EC₅₀ value, the greater the binding affinity for the antigen or cells expressing the antigen.

A kit provided by the present application comprises one or more containers containing a large number of gene constructs encoding the polypeptides of the present application and pharmaceutically acceptable excipients. The kit may also comprises instructions for use. The kit may also have a notice in a form prescribed by a governmental agency regulating the manufacture, use or sale of pharmaceuticals or biological products, which indicates that it has been licensed by the institution for the manufacture, use or sale of pharmaceuticals for humans.

### Research overview:

The present invention uses fully human phages for antibody screening to directly obtain fully human monoclonal antibodies. Compared to traditional hybridoma technology, it omits the difficult step of humanizing murine antibodies. Moreover, fully human antibodies have a lower immunogenicity than humanized murine antibodies, and have a better potential in the application of antibody drugs (including monoclonal antibodies, bispecific antibodies, ADC, etc.), cell therapy drugs (including CAR-T, CAR-NK, etc.) and detection reagents.

The present invention uses the method of antigenic protein panning, which can efficiently enrich antibodies that simultaneously bind to recombinant CD5 and the structurally natural CD5 on the cell membrane, greatly reducing the difficulty of later antibody screening and improving efficiency.

We used a large-capacity phage antibody library to screen fully human CD5-specific antibodies, and evaluated the specificity of these antibodies at the phage level by ELISA and FACS experiments. Finally, we obtained some fully human antibody clones with good specificity.

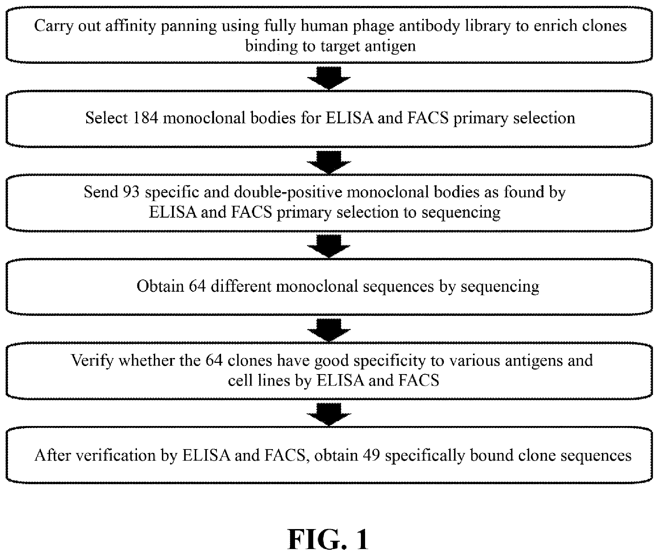

We used different antibody libraries, and after recombinant CD5 protein panning, a total of 184 monoclonal bodies were selected for primary screening by enzyme-linked immunosorbent assay (ELISA) and flow cytometry (FACS), among which 93 clones specifically bound to CD5- Fc-Bio protein and CD5 expression positive cells Jurkat, but not bound to control protein CD19-Fc-Bio and CD5 expression negative cells Raji. After sequencing, 64 different monoclonal sequences were obtained. Subsequently, we identified these 64 antibodies by flow cytometry (FACS) with various CD5 positive (Jurkat, CCRF-CEM) and negative cell lines (Raji, NALM6), and by enzyme-linked immunosorbent assay (ELISA) with CD5 proteins from different companies (Kactus-CD5- Fc-Bio, Acro-CD5-his, SB-CD5-his-Bio), non-related proteins (Kactus-BAFFR-his-Bio, Kactus-CD19-FC-Bio, SA), of which 49 clones showed good binding and specificity to multiple cell lines and various protein antigens. The acquisition of these clones lays the foundation for the subsequent development of fully human CD5 CAR-T products or antibody drugs. The overall project process is shown in Fig. 1.

The present invention will be described in detail below in conjunction with specific examples.

### Example 1. Enrichment of specific antibody clones targeting CD5 protein from phage antibody library by affinity panning

Appropriate negative panning and positive panning strategies were used to enrich the specific antibody clones we need from the phage antibody library.

### Construction of phage antibody library

The phage antibody libraries we constructed include natural libraries, semi-synthetic libraries and single-domain libraries. The semi-synthetic phage antibody library, used together with the natural library, solves the problem that the natural library may lack CD5 high-affinity antibody clones. Single-domain phage antibody library refers to an antibody library composed only of the variable region amino acids of a heavy chain antibody. Its molecular weight is only 12-15 kDa, but it has similar or higher specificity and affinity than traditional antibodies. In addition, single-domain antibodies have attracted much attention because of their stable physical and chemical properties, high affinity, easy recombinant expression and preparation, and easy combination with other target or epitope antibodies.

### CD5 protein panning

Using CD5-Fc-Bio as the positive panning protein and CD19-Fc-Bio as the negative panning protein, multiple rounds of panning were performed to obtain a phage pool enriched in target antibody clones. The experimental steps are briefly described as follows:
1) Block the SA magnetic beads with blocking buffer for 2 h, and then bind the target antigen (CD5-Fc-Bio) to the blocked SA magnetic beads;
2) Add a phage library (containing 5×10¹² phage particles) to incubate with the control antigen to deduct phage antibody clones that do not specifically bind to the Fc tag;
3) After incubation, transfer the supernatant to SA magnetic beads bound to the target antigen, and continue to incubate to allow the phage to bind to the target antigen;
4) Wash the magnetic beads with wash buffer to wash away unbound phage;
5) elute the positive phage from the target antigen using an eluent, and add a neutralizing solution for neutralizing;
6) Re-infect the host strain XL1-blue with the eluted phages, and amplify the recovered phages. Leave a small amount of sample for gradient dilution, infect the host bacteria, apply it onto Amp resistance plates, and calculate the number of recovered phages;
7) Repeat steps 1) to 6), wherein usually three rounds of panning are required until a significant increase in the recovery rate of phages (number of eluted phages or number of input phages) is observed.

The enriched phage pool can be used for subsequent monoclonal body selection and ELISA/FACS screening.

### Main materials and reagents:

Fully human phage antibody library, including natural library, semi-synthetic library and single-domain library;
Helper phage KO7, Thermo/Invitrogen, 18311019;
Recombinant biotinylated Human CD5-Fc Protein, Kactus, CD5-HM401;
Recombinant biotinylated Human CD19 Protein-Fc, Kactus, CD2-HE121
BeaverBeads^{™} Streptavidin, Beaver Bio, 22307-10;
High binding ELSIA plate, Costar, #3590
Blocking buffer: PBS+3% BSA
Wash buffer: PBS+ 0.1% Tween20
Eluent: 0.2M Glycine, pH2.2
Neutralizing solution: 1M Tris, pH9.1

### Experimental results:

Using different antibody libraries, through 3 rounds of protein panning, a significant increase in the recovery rate was observed in each panning (Table 1), proving that the antibody clones were effectively enriched.

**Table 1 Results of protein panning experiments**

| Antibody library | Round | Recovery rate | Enrichment factor |
|---|---|---|---|
| XL-SD-1 | 1st | 3.34E-05 | / |
| | 2nd | 7.74E-05 | 2.32 |
| | 3rd | 4.54E-03 | 58.66 |
| XI,-NVH | 1st | 4.54E-05 | / |
| | 2nd | 7.46E-05 | 1.64 |
| | 3rd | 1.20E-03 | 16.09 |
| XL-V1 | 1st | 4.14E-05 | / |
| | 2nd | 3.46E-05 | 0.84 |
| | 3rd | 1.20E-03 | 34.68 |
| XL-V2 | 1st | 3.60E-05 | / |
| | 2nd | 4.80E-05 | 1.33 |
| | 3rd | 2.06E-03 | 42.92 |

It can be seen that after three rounds of panning, different antibody libraries were enriched (the recovery rate of the third round was significantly higher than that of the previous round).

### Example 2. Screening of specific clones from enriched phage pools by enzyme-linked immunosorbent assay (ELISA) and flow cytometry (FACS)

Purpose and principle: The phage pool enriched by the affinity panning step contains phage antibodies of various properties: specific clones, non-specific clones, and negative clones. In order to obtain specific clones, we need to isolate monoclonal bodies from them, package them into monoclonal phages, and conduct preliminary screening on a large number of monoclonal bodies by enzyme-linked immunoassay (ELISA) and flow cytometry (FACS) to select monoclonal bodies that simultaneously specifically bind to CD5 protein and CD5 positive cell line Jurkat. The specific monoclonal bodies are further subjected to DNA sequencing to determine the unique antibody sequence contained therein.

In the ELISA primary screening, through the binding of streptavidin to biotin, the biotinylated target protein (CD5-Fc-Bio) is closer to the natural antigen conformation in the reaction solution. Those that only bind to CD5-Fc-Bio but not to the control antigen CD 19-Fc-Bio are identified as specific clones. The FACS primary screening is carried out using the positive cell line Jurkat with high expression of CD5 and the cell line Raji negative for CD5, and those that only bind to Jurkat cells but not to Raji cells are identified as specific clones. Through the two primary screenings by ELISA and FACS, we can obtain candidate antibodies that can not only bind to the recombinantly expressed CD5 protein, but also recognize the natural CD5 molecule on the cell surface, for subsequent further screening.

Brief steps of ELISA primary screening experiment:
1) Culture and package monoclonal phages in a deep-well 96-well plate;
2) Dilute streptavidin with PBS to 2 µg/mL, add the dilution at 100 µL/well to a high-binding ELISA plate for binding at room temperature for 2 h;
3) Discard the coating buffer, add 250 µL of blocking buffer to each well for blocking overnight at 4°C;
4) Wash the plate twice with 250 µL of wash buffer;
5) Dilute the biotin-labeled target protein and control protein to 2 µg/mL with PBS, add the dilution at 100 µg/well to the ELISA plate pre-coated with Streptavidin for binding for 1 h at room temperature;
6) Wash the plate twice with 250 µL of wash buffer;
7) Add 100 µL of the phage supernatant cultured in step 1) to the wells coated with the target antigen for binding at room temperature for 2 h;
8) Wash the plate 4 times with 250 µL of wash buffer;
9) Add 1:2000 diluted mouse anti M13 primary antibody, 100 µL/well, incubate at room temperature for 45 min;
10) Wash the plate 4 times with 250 µL of wash buffer;
11) Add 1:2000 diluted HRP Donkey anti-mouse IgG, 100 µL/well, incubate at room temperature for 45 min;
12) Wash the plate 6 times with 250 µL of wash buffer;
13) Add 100 µL of TMB chromogenic substrate, develop color for 5 to 10 min;
14) Add 100 µL of 2M H₂SO₄ to terminate the reaction, and read the result on a microplate reader.

Brief steps of FACS primary screening experiment:
1) Culture and package monoclonal phages in a deep-well 96-well plate;
2) Wash Raji and Jurkat cells twice with PBS, resuspend the cells in PBS at a concentration of 1×10⁷/mL, and dispense the suspended cells into 96-well deep-well plates at 50 µL;
3) Add 50 µL of packaged monoclonal phage to each well, mix well, and bind at 4°C for 2 h;
4) Wash twice with 200 µL of PBS;
5) Add mouse anti M13 primary antibody diluted at 1:2000, 100 µL/well, mix well by pipetting, and then incubate at room temperature for 45 min;
6) Wash twice with 200 µL of PBS;
7) Add 1:300 diluted FITC horse anti mouse-IgG (H+L), 100 µL/well, mix well by pipetting, and incubate at room temperature for 45 min;
8) Wash twice with 200 µL of PBS; finally resuspend the cells with 200 µL of PBS;
9) Detect the fluorescence intensity of the FITC channel of the sample on a flow cytometer, and analyze the results.

### Main materials and reagents:

Helper phage KO7, Thermo/Invitrogen, 18311019
Streptavidin, Pierce, 21125
Recombinant biotinylated Human CD5 Protein, Kactus, CD5-HM401;
Recombinant biotinylated Human CD19 Protein, Kactus, CD2-HE121
High binding ELSIA plate, Costar, #3590
Corning 96 Well Clear Round Bottom TC-Treated Microplate, Costar, #3799
Blocking buffer: PBS+3% BSA
Wash buffer: PBS+ 0.1% Tween20
Soluble one-component TMB substrate solution, Tiangen, PA-107-02
Anti-M13 Bacteriophage Coat Protein g8p antibody, abcam, ab9225
HRP Goat anti-mouse IgG (minimal x-reactivity) Antibody, Biolegend, 405306
FITC horse anti mouse-IgG (H+L), Vector, FI2000

### Experimental results:

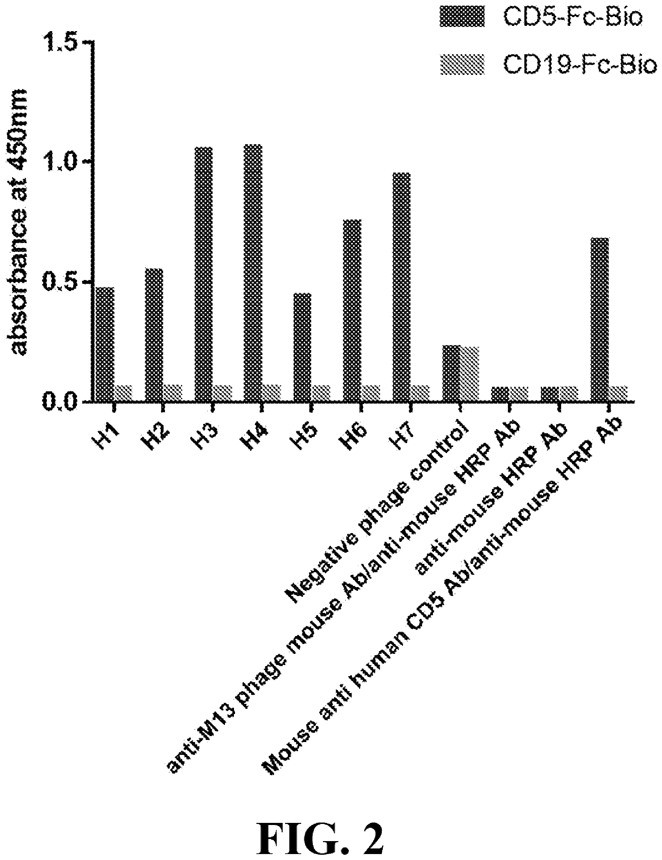

Monoclonal bodies were randomly selected from the enriched phage antibody pool, packaged into phages, and then detected for the binding of monoclonal phages to CD5-Fc-Bio protein and control protein CD19-Fc-Bio by phage ELISA to find CD5-specific phage antibody clones. The ELISA results of some clones are shown in Fig. 2. It can be seen from the figure that clones H1, H2, H3, H4, H5, H6 and H7 bound strongly to the target antigen CD5 (CD5-Fc-Bio), and not to the control antigen CD19-Fc-Bio, showing good specificity. Negative phage control was a negative control phage antibody clone, which did not bind to neither the target antigen nor control antigen; anti-M13 phage mouse Ab/anti-mouse HRP Ab was a negative antibody control with only primary and secondary antibodies added; anti-mouse HRP Ab was the negative antibody control with only the secondary antibody, they bound to neither of the target antigen nor the control antigen; mouse anti human CD5 Ab/anti-mouse HRP Ab was the positive antibody control of the target antigen (CD5-Fc-Bio), which bound to the target antigen and not to the control antigen.

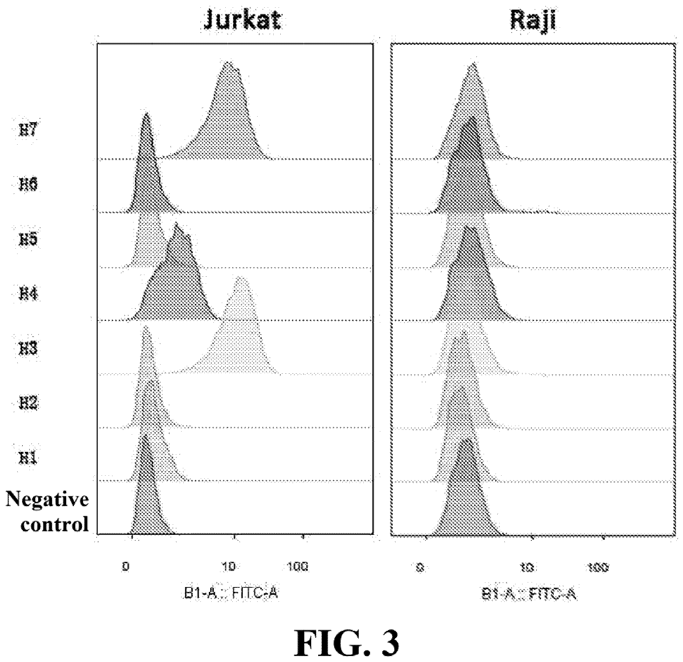

The results of FACS primary screening of antibody clones corresponding to ELISA are shown in Fig. 3. Among others, H3, H4 and H7 clones bound to Jurkat, but not to Raji cells, and were specific clones; other clones were negative clones (bound to neither of the two kinds of cells).

Through ELISA detection and FACS primary screening, we obtained a total of 93 ELISA and FACS double-positive clones with good specificity, and then we sequenced the 93 double-positive clones with good specificity, and obtained 64 different monoclonal sequences after sequencing. Then these 64 monoclonal bodies with different sequences were further tested for the binding specificity of the candidate clones through FACS identification with multiple cell lines and ELISA identification with various antigens.

### Example 3. Identification of monoclonal specificity by FACS using multiple cell lines

The purpose and principle of the experiment: the antibody used for treatment must have very good target specificity, and only bind to the target antigen, not to any unrelated antigen; on the other hand, the amino acid sequence of the same antigen on different cell lines will be different (isomers or mutants) or bind to different ligands, and it is also necessary to investigate whether our antibodies can bind to cells positive for various target proteins. In order to further analyze the specificity and universality of these monoclonal bodies and find the best candidate clones, we further evaluated the specificity of the primarily screened clones by flow cytometry. In this experiment, we used a variety of CD5-positive cell lines and a variety of CD5-negative cell lines to react with these monoclonal phage antibodies to analyze whether these clones can bind to CD5 antigen on different cell lines, and whether they have any non-specific binding to other cell lines that do not express CD5. Through this experiment, we obtained several clones with excellent specificity.

Experimental method: the same as the FACS primary screening;

### Main samples and reagents:

Jurkat cell line, CD5 positive cell line;
Raji cell line, CD5 negative cell line;
CCRF-CEM cell line, CD5 positive cell line;
NALM6 cell line, CD5 negative cell line;
The rest of the reagents were the same as the FACS primary screening.

### Experimental results:

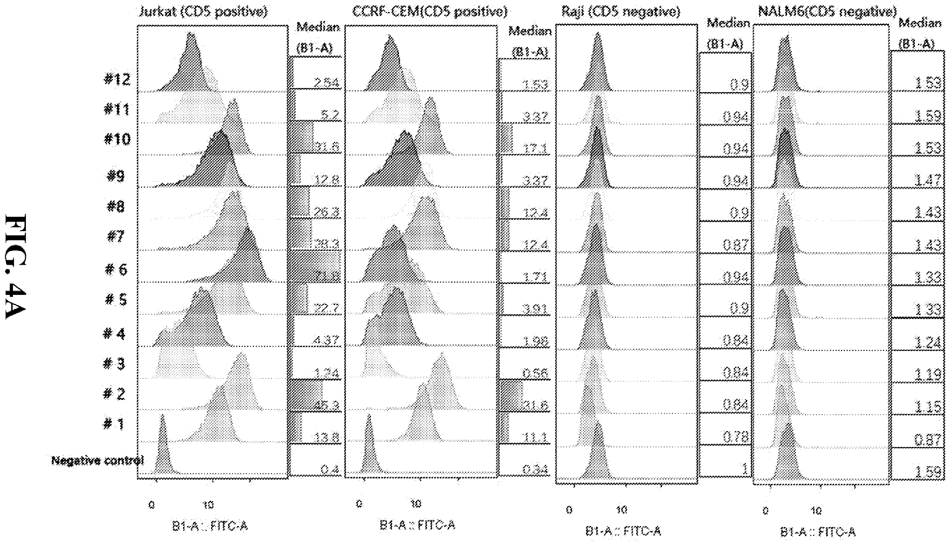

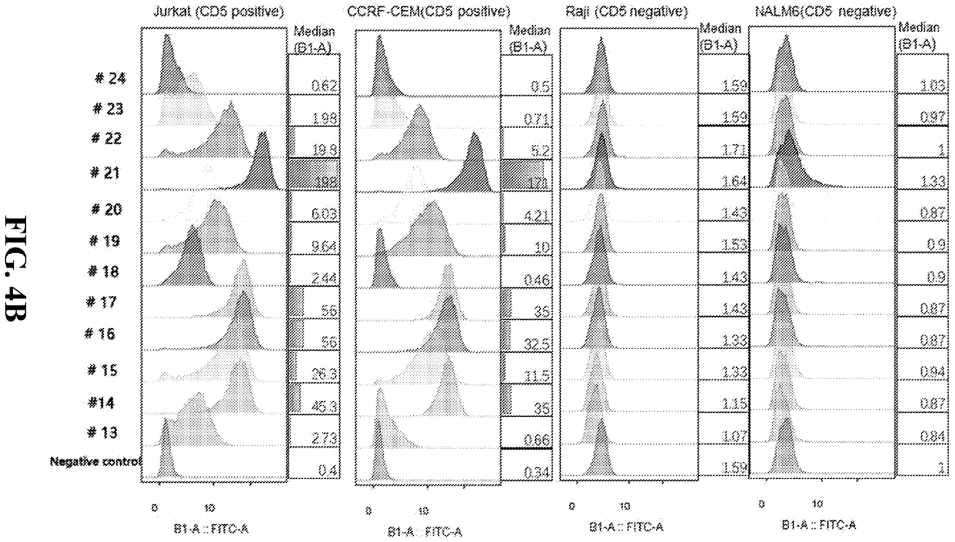

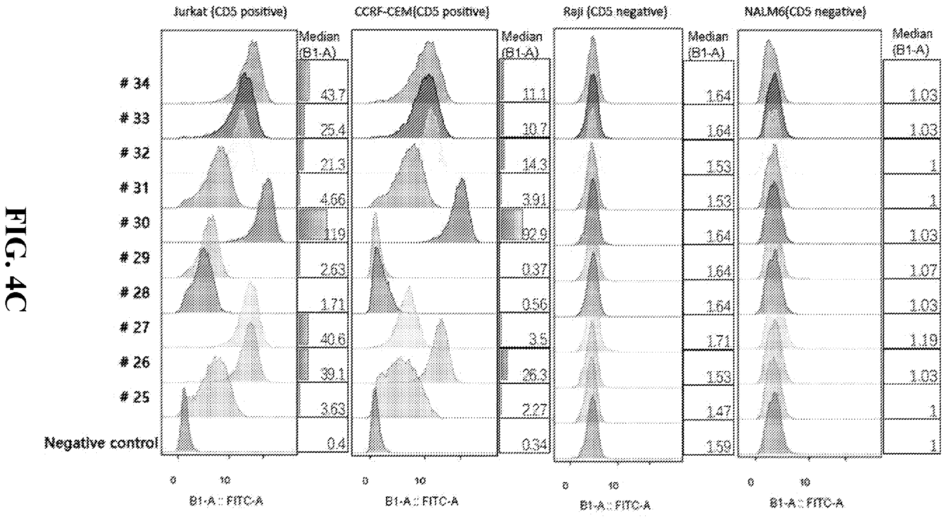

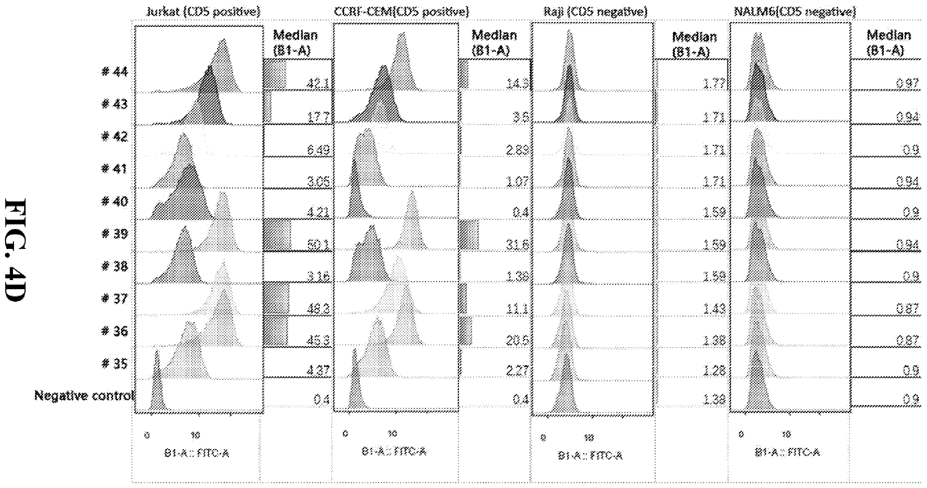

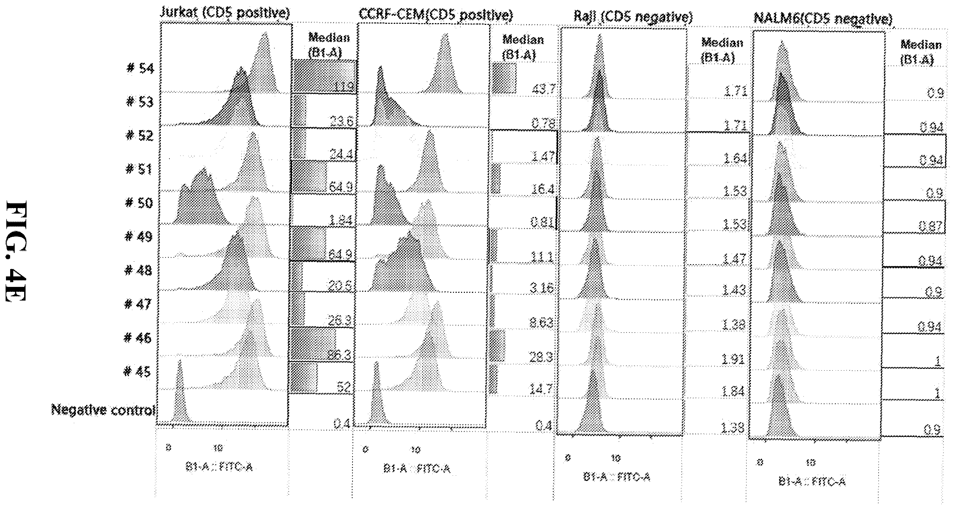

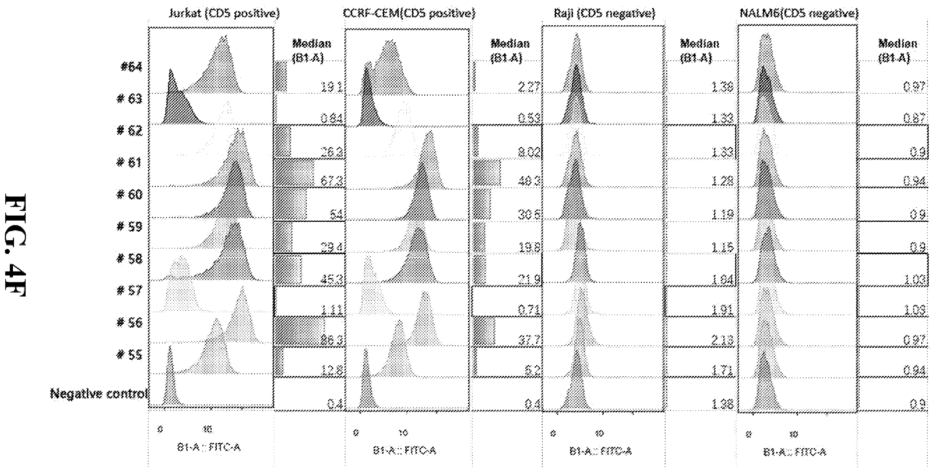

Antibodies used for therapy must have very good target specificity. In order to further analyze the specificity of these monoclonal antibodies, we identified the unique specific clone obtained in Example 2 with more antigens and cell lines by using ELISA and flow cytometry. The results are shown in Figs. 4A-F, wherein Negative Control is the negative control phage antibody clone. Clones #1-2, #4-12, #14-17, #19-22, #25-27, #30-39, #42-49, #51, #54-55, #58-62, #64 bound to both of the two CD5-positive cell lines Jurkat and CCRF-CEM with strong or weak median fluorescence intensity (MFI), and bound to neither of the two CD5-negative cell lines Raji and NALM6 with low MFI, showing good specificity. Clones #3, #13, #18, #23, #24, #28, #29, #40, #41, #50, #57 and #63 weakly bound to or did not bind to the positive cell line Jurkat, and did not bind to the positive cell line CCRF-CEM, so that they are negative clones; clones 52 and 53 only bound to the positive cell line Jurkat, but not to CCRF-CEM, indicating that they cannot recognize CD5 antigens of different conformations or isomers expressed by different cell lines and do not meet the experimental requirements; clone 56 could bind to both of the two positive cell lines, but had weaker binding to the negative cell line Raji, indicating that its binding may be non-specific and does not meet the experimental requirements.

### Example 4. Identification of monoclonal specificity by ELISA using antigens from different companies

The purpose and principle of the experiment: the antibody used for treatment must have very good target specificity, and only bind to the target antigen, not to any unrelated antigen; on the other hand, the amino acid sequence of the same antigen produced by different companies will be different (isomers or mutants), and it is also necessary to investigate whether our antibodies can bind to various target proteins. In order to further analyze the specificity and universality of these monoclonal bodies and find the best candidate clones, we further evaluated the specificity of the primarily screened clones by enzyme-linked immunosorbent assay (ELISA). In this experiment, we used CD5 antigens purchased from different companies and various CD5-unrelated antigens to react with these monoclonal phage antibodies, and analyzed whether these clones could bind to different CD5 antigens and whether they had any non-specific binding to other CD5-unrelated antigens. Through this experiment, we obtained several clones with excellent specificity.

Experimental method: the same as the ELISA primary screening;

### Main samples and reagents:

| **Abbreviation** | **Name** | **Manufacturer** | **Item No.** |
|---|---|---|---|
| Acro-CD5-his | Human CD5 Protein, his tag | ACRObiosystem | CD5-H52H5 |
| Kactus-CD5-Fc-Bio | Recombinant biotinylated Human CD5 Protein | Kactus | CD5-HM401 |
| SB-CD5-his-Bio | SB-human CD5 Protein, his tag, Bio | Sino Biological | 11027-H27H-B |
| Kactus-BAFFR-his-Bio | Biotinylated Recombinant human BAFFR Protein | Kactus | BAF-HM40RB |
| Kactus-CD19-FC - Bio | Recombinant biotinylated Human CD 19 Protein | Kactus | CD2-HE121 |
| SA | Streptavidin, | Pierce | 21125 |
| Mouse anti-human CD5 Ab | APC-mouse anti-human CD5 | BD | 555355 |

The rest of the reagents were the same as the ELISA primary screening.

### Experimental results:

Antibodies used for therapy must have very good target specificity. In order to further analyze the specificity of these monoclonal antibodies, we identified the multiple clones obtained in Example 2 on various antigens using enzyme-linked immunosorbent assay (ELISA). The results are shown Figs. 5A-F. Negative control was a negative control phage antibody clone, which did not bind to the target antigen or control antigen; anti-M13 phage mouse Ab/anti-mouse HRP Ab was a negative antibody control with only primary and secondary antibodies added; anti-mouse HRP Ab was the negative antibody control with only the secondary antibody, they bound to neither of the target antigen and the control antigen; mouse anti human CD5 Ab/anti-mouse HRP Ab was the positive antibody control of the target antigen (CD5-Fc-Bio), which bound to the target antigen and not to the control antigen. Anti-human IgG-HRP Ab/anti-his-HRP Ab was a positive antibody control for detecting antigen tags, which binds to antigens containing Fc tags or his tags, indicating that the coated antigens have been bound to the ELISA plate. Clones #1-64 bound to all three CD5 antigens, but to none of the three non-related antigens, indicating that they can bind to CD5 antigens from different companies with good specificity. Among them, clones #42, #60, #61 and #62 are single-domain antibodies, and their CDR sequences are as follows:

| Clone ID | HCDR1 | HCDR2 | HCDR3 |
|---|---|---|---|
| #42 | GFTFSHSA (SEQ ID NO: 1) | IYARGGYT (SEQ ID NO: 2) | ARGYHLEYMVSQDV (SEQ ID NO: 3) |
| #60 | GFTFSSYE (SEQ ID NO: 4) | ISSSGSTI (SEQ ID NO: 5) | ARVAQREGDV (SEQ ID NO: 6) |
| #61 | GGTFSNYA (SEQ ID NO: 7) | ISAYNGDT (SEQ ID NO: 8) | ARYESMSGQDI (SEQ ID NO: 9) |
| #62 | GYSFSNHW (SEQ ID NO: 10) | VYPGDSDT (SEQ ID NO: 11) | ARGGTIDGDYGGRQDF (SEQ ID NO: 12) |

### Example 5. Determination of the affinity of anti-CD5 sdAbs

### Experimental purpose and principle:

The affinity between CD5 sdAbs and the antigen may have an important impact on the killing effect and duration of CAR-T in patients. In order to determine this important property, we used ForteBio's Octet molecular interaction technology to measure it. The biolayer interferometry used in the Octet system is a label-free technology that provides high-throughput biomolecular interaction information in real time. The instrument emits white light to the surface of the sensor and collects the reflected light. The reflected light spectra of different frequencies are affected by the thickness of the optical film layer of the biosensor. Some frequencies of reflected light form constructive interference (blue), while others are affected by destructive interference (red). These interferences are detected by the spectrometer to form an interference spectrum, which is displayed as the phase shift intensity (nm) of the interference spectrum. Therefore, once the number of molecules bound to the sensor surface increases or decreases, the spectrometer will detect the shift of the interference spectrum in real time, and this shift directly reflects the thickness of the biofilm on the sensor surface, from which high-quality data of the biomolecular interaction can be obtained, so as to determine the kinetic parameters of biomolecular interactions (Kon, Kdis and KD), providing important information for the research and development process.

Brief experimental steps:
1) Dilute anti-CD5 IgG (formed by fusing the VHH sequence of CD5 with human IgG4 Fc) to 20 µg/mL with loading buffer (1×PBS, pH 7.4, 0.01% BSA and 0.02% Tween 20), and load it onto a biosensor at about 0.8 nM.
2) After a 60s equilibration period, monitor the binding kinetics of the CD5 antigen (Aero, CD5-H52H5) at various antigen concentrations (100 to 1.563 nM). At each concentration, carry out 160s binding and 300s dissociation respectively.
3) Wash 3 times with 10mM Glycine-HCl, pH1.5 to regenerate the chip.
4) Analyze the binding constant by using a 1:1 binding site model (Biacore X-100 evaluation software).

### Experimental results:

Affinity refers to the strength of the binding of a single molecule to its ligand and is usually measured and reported by the equilibrium dissociation constant (KD), and the equilibrium dissociation constant can be used to assess and rank the strength of the interaction between two molecules. The binding of an antibody to its antigen is a reversible process, and the rate of the binding reaction is directly proportional to the concentration of the reactants. The smaller the KD value, the greater the affinity of the antibody for its target. As shown in Table 2: #42, #61, #61-42 (#61 and #42 connected by linker (GGGGSGGGGSGGGGS) (SEQ ID NO: 21)), #62 and #60 can all bind to CD5 antigen, and the affinity of #61-42 is slightly higher than that of #42, #61, #62 and #60.

**Table 2 Anti-CD5 IgG affinity determination**

| Analyte | KD (M) | kon (1/Ms) | kdis (1/s) |
|---|---|---|---|
| #42 IgG | 2.90E-09 | 4.80E+04 | 1.39E-04 |
| #61 IgG | 3.96E-09 | 7.28E+04 | 2.88E-04 |
| #61-42 IgG | 1.67E-09 | 1.13E+05 | 1.89E-04 |
| #62 IgG | 2.74E-08 | 1.25E+04 | 3.09E-04 |
| #60 IgG | 8.32E-09 | 2.55E+04 | 2.12E-04 |

### Example 6. Study on binding of tandem single-domain antibody to CD5⁺ target cells

Research purpose: To investigate the binding ability of CD5 single-domain antibody to CD5⁺ target cells.

Research method: After CD5⁺ target cells were incubated with different concentrations of CD5 tandem single-domain antibody rabbit Fc fusion protein 61-42-rFc, the cells were washed twice with PBS, then fluorescent dye-labeled rabbit Fc antibody was added to mark positive cells, and FCM (flow cytometry) was used for detection. By analyzing the relationship between the percentage of fluorescently labeled positive CD5⁺ target cells and different concentrations of the CD5 tandem single-domain antibodies, the EC50 constant was calculated by fitting using Graphpad Prism software. This experiment was repeated three times in total independently.

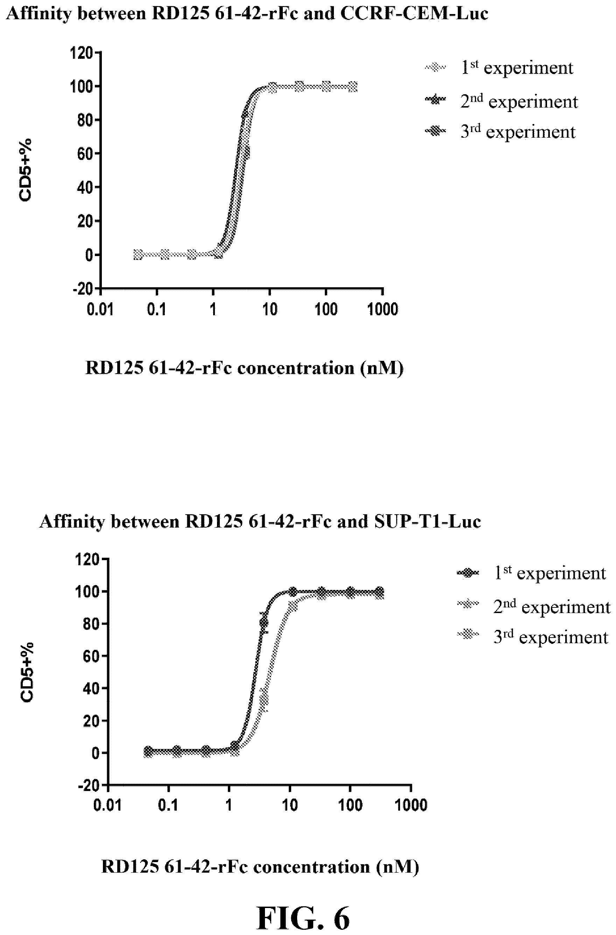

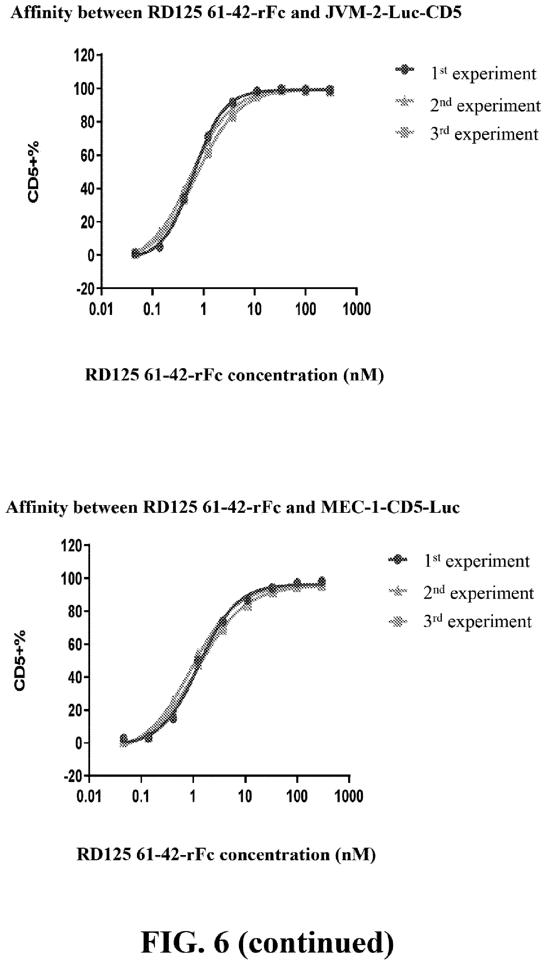

Research results: There was high affinity between the CD5 tandem single-domain antibody rabbit FC fusion protein 61-42 rFc (formed by fusing the above #61-42 with rFc) and the four strains of CD5 positive cells, with Kd being: 2.99± 0.35nM (CCRF-CEM-Luc); 4.02±0.92nM (SUP-T1-Luc); 0.64±0.07nM (JVM-2-Luc-CDS); 1.14±0.16nM (MEC-1-CDS-Luc). The specific results are shown in Table 3 and Fig. 6.

**Table 3 Summary of the affinity between 61-42-rFc single-domain antibody and CD5 positive cells**

| **Name** | **Cell name** | **Number of experiments** | **Apparent affinity** | **Average (nM)** | **Standard deviation** |
|---|---|---|---|---|---|
| 61-42-rFc | CCRF-CEM-Luc | 1st | 3.04 | 2.99 | 0.35 |
| | | 2nd | 2.54 | | |
| | | 3rd | 3.39 | | |
| | SUP-T1-Luc | 1st | 2.72 | 4.02 | 0.92 |
| | | 2nd | 4.58 | | |
| | | 3rd | 4.75 | | |
| | JVM-2-Luc-CD5 | 1st | 0.64 | 0.64 | 0.07 |
| | | 2nd | 0.56 | | |
| | | 3rd | 0.72 | | |
| | MEC-1-CD5-Luc | 1st | 1.30 | 1.14 | 0.16 |
| | | 2nd | 0.92 | | |
| | | 3rd | 1.20 | | |

Research conclusion: The CD5 tandem single-domain antibody rabbit Fc fusion protein 61-42-rFc has stable, good and specific binding ability to the 4 strains of CD5 positive cells tested, and the EC50 values are all in the range of 1-5 nM.

### References:

1. Voisinne G, Gonzalez de Peredo A, Roncagalli R. CD5, an Undercover Regulator of TCR Signaling. Front Immunol. 2018;9:2900. Published 2018 Dec 7. doi:10.3389/fimmu.2018.02900.
2. Bamberger M, Santos AM, Goncalves CM, et al. A new pathway of CD5 glycoprotein-mediated T cell inhibition dependent on inhibitory phosphorylation of Fyn kinase. J Biol Chem. 2011;286(35):30324-30336. doi:10.1074/jbc.M111.230102.
3. Perez-Villar JJ, Whitney GS, Bowen MA, Hewgill DH, Aruffo AA, Kanner SB. CD5 negatively regulates the T-cell antigen receptor signal transduction pathway: involvement of SH2-containing phosphotyrosine phosphatase SHP-1. Mol Cell Biol. 1999;19(4):2903-12.
4. Gary-Gouy H, Sainz-Perez A, Marteau JB, et al. Natural phosphorylation of CD5 in chronic lymphocytic leukemia B cells and analysis of CDS-regulated genes in a B cell line suggest a role for CD5 in malignant phenotype. J Immunol. 2007;179(7):4335-4344. doi:10.4049/jimmunol.179.7.4335
5. Freitas CMT, Johnson DK, Weber KS. T Cell Calcium Signaling Regulation by the Co-Receptor CD5. Int J Mol Sci. 2018;19(5):1295. Published 2018 Apr 26. doi:10.3390/ijms 19051295
6. Pui CH, Behm FG, Crist WM. Clinical and biological relevance of immunological marker studies in childhood acute lymphoblastic leukemia. Blood. 1993;82(2):343-362.
7. Campana D, van Dongen JJ, Mehta A, et al. Stages of T-cell receptor protein expression in T-cell acute lymphoblastic leukemia. Blood. 1991;77(7):1546-1554.
8. Huang H, Li Z, Huang C, et al. CD5 and CD43 Expression are Associate with Poor Prognosis in DLBCL Patients. Open Med (Wars). 2018;13:605-609. Published 2018 Nov 27. doi:10.1515/med-2018-0089
9. Friedman DR, Guadalupe E, Volkheimer A, Moore JO, Weinberg JB. Clinical outcomes in chronic lymphocytic leukaemia associated with expression of CD5, a negative regulator of B-cell receptor signaling. Br J Haematol. 2018;183(5):747-754. doi:10.1111/bjh.15632
10. Dillman RO, Shawler DL, Dillman JB, Royston I. Therapy of chronic lymphocytic leukemia and cutaneous T-cell lymphoma with T101 monoclonal antibody. J Clin Oncol. 1984;2(8):881-891. doi:10.1200/JCO.1984.2.8.881
11. Foss FM, Raubitscheck A, Mulshine JL, et al. Phase I study of the pharmacokinetics of a radioimmunoconjugate, 90Y-T101, in patients with CDS-expressing leukemia and lymphoma. Clin Cancer Res. 1998;4(11):2691-2700.
12. Phage display-methods and protocols, ISSN 1064-3745 ISSN 1940-6029 (electronic) Methods in Molecular Biology ISBN 978-1-4939-7446-7 ISBN 978-1-4939-7447-4 (eBook), DOI 10.100 7/978 -1-4939-7447-4.

SEQ ID NO:13 #42 VH DNA sequence: 363 bp 
GAAGTTCAGCTGCTGGAAAGCGGTGGTGGTCTGGTTCAGCCTGGTGGTAGCCTGC
GTCTGAGCTGTGCAGCAAGCGGTTTTACCTTTAGCCATAGCGCCATGGGTTGGGTTC
GTCAGGCACCTGGTAAAGGTCTGGAATGGGTTAGCAGCATCTATGCCCGCGGCGGCT
ATACCTATTATGCAGATAGCGTTAAAGGTCGTTTTACCATTAGCCGTGATAACAGCA
AAAATACCCTGTACCTGCAGATGAATAGTCTGCGTGCAGAGGATACCGCAGTGTATT
ATTGTGCGCGCGGTTACCATCTGGAATACATGGTTTCTCAGGATGTTTGGGGTCAAG
GTACTCTGGTGACCGTCTCCTCA
SEQ ID NO: 14 #60 VH DNA sequence: 351 bp 
CAGGTGCAGCTGGTGGAGTCTGGGGGAGGCTTGGTACAGCCTGGAGGGTCCCTG
AGACTCTCCTGTGCAGCCTCTGGATTCACCTTCAGTAGTTATGAAATGAACTGGGTC
CGCCAGGCTCCAGGGAAGGGGCTGGAGTGGGTTTCATACATTAGTAGTAGTGGTAGT
ACCATATACTACGCAGACTCTGTGAAGGGCCGATTCACCATCTCCAGAGACAACGCC
AAGAACTCACTGTATCTGCAAATGAACAGTCTGAGAGCCGAGGACACGGCTGTTTAT
TACTGTGCGAGAGTCGCCCAGAGAGAAGGGGACGTCTGGGGCAAAGGGACCACGGT
CACCGTCTCCTCA
SEQ ID NO: 15 #61 VH DNA sequence: 354 bp 
CAGGTGCAGCTGGTGCAGTCTGGGGCTGAGGTGAAGAAGCCTGGGTCCTCGGTG
AAGGTCTCCTGCAAGGCTTCTGGAGGCACCTTCAGCAACTATGCTATCAGCTGGGTG
CGACAGGCCCCTGGACAAGGGCTTGAGTGGATGGGATGGATCAGCGCCTACAATGG
TGACACAAAATATGCACAGAGGCTCCAGGGCAGAGTCACCATGACCACAGACACAT
CCACGAGCACAGCCTACATGGAGCTGAGGAACCTAAGATCTGACGACACGGCCGTG
TATTACTGTGCGCGCTACGAATCTATGTCTGGTCAGGATATCTGGGGTCAAGGTACT
CTGGTGACCGTCTCCTCA
SEQ ID NO: 16 #62 VH DNA sequence: 369 bp 
CAGGTCCAGCTGGTGCAGTCTGGGGCTGAGGTGAAAAAGCCCGGGGAGTCTCTG
AGGATCTCCTGTAAGGCTTCTGGATACAGTTTTAGCAACCATTGGATCGGCTGGGTG
CGCCAGAAGCCCGGGAAAGGCCTGGAGTGGATGGGGAGCGTCTATCCTGGTGACTC
CGATACCAGATACAGCCCGTCCTTCCAAGGCCACGTCACTGTCTCAGCCGACAAGTC
CATGAATACCGCCTACCTGCAGTGGAGCAGCCTGAAGACCTCGGACACCGCCATGT
ATTATTGTGCGAGAGGTGGGACTATTGACGGTGACTACGGGGGGAGGCAAGACTTC
TGGGGCCAGGGAACCATGGTCACCGTCTCTTCA
SEQ ID NO: 17 #42 VH amino acid sequence: 121 aa 
EVQLLESGGGLVQPGGSLRLSCAASGFTFSHSAMGWVRQAPGKGLEWVSSIYARGG
YTYYADSVKGRFTISRDNSKNTLYLQMNSLRAEDTAVYYCARGYHLEYMVSQDVWGQ
GTLVTVSS
SEQ ID NO: 18 #60 VH amino acid sequence: 117 aa 
QVQLVESGGGLVQPGGSLRLSCAASGFTFSSYEMNWVRQAPGKGLEWVSYISSSGST
IYYADSVKGRFTISRDNAKNSLYLQMNSLRAEDTAVYYCARVAQREGDVWGKGTTVT
VSS
SEQ ID NO: 19 #61 VH amino acid sequence: 118 aa 
QVQLVQSGAEVKKPGSSVKVSCKASGGTFSNYAISWVRQAPGQGLEWMGWISAYN
GDTKYAQRLQGRVTMTTDTSTSTAYMELRNLRSDDTAVYYCARYESMSGQDIWGQGT
LVTVSS
SEQ ID NO: 20 #62 VH amino acid sequence: 123 aa

QVQLVQSGAEVKKPGESLRISCKASGYSFSNHWIGWVRQKPGKGLEWMGSVYPGD
SDTRYSPSFQGHVTVSADKSMNTAYLQWSSLKTSDTAMYYCARGGTIDGDYGGRQDF
WGQGTMVTVSS

## Claims

1. A CDS-targeting antibody or an antigen-binding fragment thereof, wherein the antibody comprises a heavy chain variable region (HCVR), the heavy chain variable region comprises HCDR1, HCDR2 and HCDR3, and the HCDR1, HCDR2 and HCDR3 are selected from one of the following combinations:
(1) the amino acid sequence of HCDR1 is GFTFSHSA (SEQ ID NO: 1);
the amino acid sequence of HCDR2 is IYARGGYT (SEQ ID NO: 2);
the amino acid sequence of HCDR3 is ARGYHLEYMVSQDV (SEQ ID NO: 3);
(2) the amino acid sequence of HCDR1 is GFTFSSYE (SEQ ID NO: 4);
the amino acid sequence of HCDR2 is ISSSGSTI (SEQ ID NO: 5);
the amino acid sequence of HCDR3 is ARVAQREGDV (SEQ ID NO: 6);
(3) the amino acid sequence of HCDR1 is GGTFSNYA (SEQ ID NO: 7);
the amino acid sequence of HCDR2 is ISAYNGDT (SEQ ID NO: 8);
the amino acid sequence of HCDR3 is ARYESMSGQDI (SEQ ID NO: 9);
(4) the amino acid sequence of HCDR1 is GYSFSNHW (SEQ ID NO: 10);
the amino acid sequence of HCDR2 is VYPGDSDT (SEQ ID NO: 11);
the amino acid sequence of HCDR3 is ARGGTIDGDYGGRQDF (SEQ ID NO: 12); or
the antibody comprises a variant of the combination of CDR sequences in any one of (1)-(4), wherein compared to the CDR sequences in any one of (1)-(4), the variant has at least 90% sequence identity, or comprise a total of at least 1 and no more than 10, or no more than 5, 4, 3, 2 or 1 amino acid changes in the CDR sequences.

2. The antibody or antigen-binding fragment thereof of claim 1, wherein the amino acid sequence of the heavy chain variable region is selected from any one of the following:
(1) an amino acid sequence as set forth in SEQ ID NO: 17 or a heavy chain variable region having at least 90% sequence identity therewith;
(2) an amino acid sequence as set forth in SEQ ID NO: 18 or a heavy chain variable region having at least 90% sequence identity therewith;
(3) an amino acid sequence as set forth in SEQ ID NO: 19 or a heavy chain variable region having at least 90% sequence identity therewith;
(4) an amino acid sequence as set forth in SEQ ID NO: 20 or a heavy chain variable region having at least 90% sequence identity therewith.

3. The antibody or antigen-binding fragment thereof of claim 1 or 2, wherein the amino acid sequence of the heavy chain variable region is selected from any one of the following:
(1) a heavy chain variable region sequence as set forth in SEQ ID NO: 17;
(2) a heavy chain variable region sequence as set forth in SEQ ID NO: 18;
(3) a heavy chain variable region sequence as set forth in SEQ ID NO: 19;
(4) a heavy chain variable region sequence as set forth in SEQ ID NO: 20.

4. The antibody or antigen-binding fragment thereof of any one of claims 1-3, wherein the antibody is a single-domain antibody.

5. The antibody or antigen-binding fragment thereof of any one of claims 1-4, wherein the antibody is a fully human antibody.

6. The antibody or antigen-binding fragment thereof of any one of claims 1-5, wherein the binding KD value of the antibody to the CD5 antigen measured by biolayer interferometry is lower than 10⁻⁷ M, preferably lower than 10⁻⁸M.

7. A fusion protein comprising one or two antigen-binding functional moieties, wherein each of the antigen-binding functional moieties comprises the antibody or antigen-binding fragment thereof of any one of claims 1-6; preferably, the fusion protein further comprises an Fc fragment.

8. The fusion protein of claim 7, wherein the two antigen-binding functional moieties respectively bind to the same or different antigenic epitopes.

9. The fusion protein of claim 7 or 8, comprising a first antigen-binding functional moiety and a second antigen-binding functional moiety connected in tandem; wherein the first antigen-binding functional moiety comprises a first heavy chain variable region (HCVR), and the first heavy chain variable region comprises HCDR1 as set forth in SEQ ID NO: 7, HCDR2 as set forth in SEQ ID NO: 8 and HCDR3 as set forth in SEQ ID NO: 9; wherein the second antigen binding functional moiety comprises a second heavy chain variable region (HCVR), and the second heavy chain variable region comprises HCDR1 as set forth in SEQ ID NO: 1, HCDR2 as set forth in SEQ ID NO: 2, and HCDR3 as set forth in SEQ ID NO: 3.

10. The fusion protein of any one of claims 7-9, comprising the heavy chain variable region sequence as set forth in SEQ ID NO: 19 and the heavy chain variable region sequence as set forth in SEQ ID NO: 17 connected in tandem.

11. The fusion protein of any one of claims 7-10, wherein the antigen-binding functional moieties are directly connected through a linker molecule; preferably, the linker molecule comprises an amino acid sequence as set forth in SEQ ID NO: 21.

12. The fusion protein of any one of claims 7-11, wherein the EC₅₀ value of the binding between the fusion protein and CD5 positive cells determined by flow cytometry is 1-5 nM.

13. A nucleic acid molecule encoding the antibody or antigen-binding fragment thereof of any one of claims 1-6 or the fusion protein of any one of claims 7-12.

14. The nucleic acid molecule of claim 13, comprising a nucleotide sequence as set forth in any one of SEQ ID NOs: 13-16.

15. An expression vector, comprising the nucleic acid molecule of claim 13 or 14.

16. A host cell, comprising the expression vector of claim 15.

17. A pharmaceutical composition, comprising
1) the antibody or antigen-binding fragment thereof of any one of claims 1-6 or the fusion protein of any one of claims 7-12; and
2) a pharmaceutically acceptable carrier or diluent.

18. A method of treating a disease or condition, comprising administering to a patient in need thereof a therapeutically effective amount of the antibody or antigen-binding fragment thereof of any one of claims 1-6, the fusion protein of any one of claims 7-12, the host cell of claim 16, or the pharmaceutical composition of claim 17 to eliminate, inhibit or reduce CD5 activity, thereby preventing, alleviating, ameliorating or inhibiting the disease or condition.

19. The method of claim 18, wherein the disease or condition is selected from: cancers.

20. The method of claim 19, wherein the cancer is selected from: T-cell malignancies.

21. An antibody or fragment competing for the same epitope as the antibody or antigen-binding fragment thereof of any one of claims 1-6.

22. A kit for detecting CD5 protein in a sample, wherein the kit comprises the antibody or antigen-binding fragment thereof of any one of claims 1-6, or the fusion protein of any one of claims 7-12.

23. Use of the antibody or antigen-binding fragment thereof of any one of claims 1-6, the fusion protein of any one of claims 7-12, or the host cell of claim 16 in the preparation of a drug for eliminating, inhibiting or reducing CD5 activity, thereby preventing, alleviating, ameliorating or inhibiting a disease or condition.

24. The use of claim 23, wherein the disease or condition is selected from: cancers or autoimmune diseases.

25. The use of claim 24, wherein the cancer is selected from: malignant T-cell tumors or malignant B-cell tumors.

26. The use of claim 25, wherein the malignant T-cell tumor is selected from T-cell acute lymphoblastic leukemia (T-ALL), T-cell lymphoma (TCL), and the malignant B-cell tumor is selected from chronic lymphocytic leukemia (B-CLL) or mantle cell lymphoma (B-MCL).

27. A multispecific antibody molecule, comprising at least a first functional moiety and a second functional moiety, wherein the first functional moiety comprises the antibody or antigen-binding fragment thereof of any one of claims 1-6; the second functional moiety has different binding specificity than the first functional moiety.

28. The multispecific antibody molecule of claim 27, wherein the second functional moiety has binding specificity for immune cells.

29. The multispecific antibody molecule of claim 27 or 28, wherein the second functional moiety has binding specificity for T-cells.

30. The multispecific antibody molecule of any one of claims 27-29, wherein the second functional moiety has binding specificity for CD7.

31. An immunoconjugate comprising the antibody or antigen-binding fragment thereof of any one of claims 1-6 linked to a therapeutic agent.

32. The immunoconjugate of claim 31, wherein the therapeutic agent is a drug.

33. The immunoconjugate of claim 31 or 32, wherein the therapeutic agent is a cytotoxin.

34. The immunoconjugate of any one of claims 31-33, wherein the therapeutic agent is a radioisotope.

35. Use of the antibody or antigen-binding fragment thereof of any one of claims 1-6, the fusion protein of any one of claims 7-12, the multispecific antibody molecule of any one of claims 27-30, or the immunoconjugate of any one of claims 31-34 in the preparation of a drug for eliminating, inhibiting or reducing CD5 activity.

36. The use of claim 35, wherein the drug is used for preventing, alleviating, ameliorating or inhibiting cancers or autoimmune diseases.

37. The use of claim 36, wherein the cancer is selected from: malignant T-cell tumors or malignant B-cell tumors.

38. Use of the antibody or antigen-binding fragment thereof of any one of claims 1-6, the fusion protein of any one of claims 7-12, the multispecific antibody molecule of any one of claims 27-30, or the immunoconjugate of any one of claims 31-34 in the preparation of a vaccine, preferably an antibody vaccine, and more preferably an anti-idiotypic antibody vaccine.

39. A vaccine preparation, comprising the antibody or antigen-binding fragment thereof of any one of claims 1-6, the fusion protein of any one of claims 7-12, the multispecific antibody molecule of any one of claims 27-30, or the immunoconjugate of any one of claims 31-34.
